# EUROPÄISCHE PATENTSCHRIFT

(11) **EP 1 641 381 B1**
(45) Veröffentlichungstag und Bekanntmachung des Hinweises auf die Patenterteilung: **08.05.2013**
(21) Anmeldenummer: 04740611.1
(22) Anmeldetag: 03.07.2004
(51) Int. Cl.: A61B 3/06, A61B 3/11

(54) **AUGENUNTERSUCHUNGSGERÄT UND VERFAHREN ZUR CHARAKTERISIERUNG DER SEHFUNKTION**
EYE EXAMINATION APPLIANCE AND METHOD FOR CHARACTERISING THE VISUAL FUNCTION
APPAREIL D'ANALYSE OCULAIRE ET METHODE POUR CARACTERISER LA FONCTION VISUELLE

(30) Priorität: 08.07.2003 DE 10331592
(43) Veröffentlichungstag der Anmeldung: 05.04.2006
(73) Patentinhaber: Carl Zeiss Meditec AG, 07745 Jena (DE)
(72) Erfinder: DONNERHACKE, Karl-Heinz, 07747 Jena (DE); DICK, Manfred, 07926 Gefell (DE)
(86) Internationale Anmeldenummer: PCT/EP2004/007266
(87) Internationale Veröffentlichungsnummer: WO 2005/004706

(56) Entgegenhaltungen:
- WO-A-02/083078
- CA-A- 2 438 109
- US-A- 5 963 300
- US-A1- 2002 140 902

## Beschreibung

### Stand der Technik

Als Dämmerungssehen (mesopisches Sehen) bezeichnet man den Bereich zwischen photopischen und skotopischen Sehen. Er umfasst Umgebungshelligkeiten von etwa 32 bis 0,0032 Candela pro Quadratmeter (cd/m²). Im Straßenverkehr tritt dieser Helligkeitsbereich in der Dämmerung und nachts im Scheinwerferkegel eines Kraftfahrzeuges auf.

Die visuelle Wahrnehmung ist beim Dämmerungssehen schlechter als beim Tagessehen, vor allem steigt die Schwelle des wahrnehmbaren Kontrastes. Hauptursache dafür sind die Sehfehler (Aberrationen) höherer Ordnung, die mit wachsendem Pupillendurchmesser bei sinkender Umgebungshelligkeit das Kontrastsehvermögen beeinträchtigen.

Das mesopische Kontrastsehen gehört zu den kritischen Sehleistungen vor allem im Straßenverkehr. Im Gegensatz zur Tagessehschärfe, die unter hohem Kontrast geprüft wird, spielen in der Dämmerung gröbere Strukturen mit geringem Kontrast die wichtigere Rolle. Die Messung des mesopischen Kontrastsehens ist eine komplexe Bestimmung der Sehschärfe und der Kontrastempfindlichkeit, wobei nur der Kontrast variiert wird. Die gefundene Kontrastschwelle wird als "Dämmerungssehschärfe" bezeichnet.

Die Methoden zur Bestimmung des mesopischen Kontrastsehens mit und ohne Blendung sind ursprünglich entwickelt worden, um die Sehleistungen zu überprüfen, die ein Verkehrsteilnehmer beim nächtlichen Fahren erbringen muss. Die Untersuchungen ergeben aber auch diagnostische Hinweise über die Transparenz der brechenden Medien einerseits und über die neuronalen visuellen Funktionen andererseits (Diabetische Retinopathie, AMD, Glaukom) und sind zurzeit hochaktuell für die umfassende Beurteilung der Sehqualität vor und nach refraktiv-chirurgischen Eingriffen.

Bei den üblichen Geräten zur Prüfung des mesopischen Kontrastsehvermögens (Dämmerungssehen) und der Blendungsempfindlichkeit wie Kontrastometer (Hersteller BKG Medizintechnik, Bayreuth), Mesotest II (Hersteller Oculus Optikgeräte, Wetzlar) und Nyktometer 500 (Hersteller Rodenstock Instrumente, Ottobrunn) werden bei definierten Umfeldbeleuchtungen (für photopisches Sehen bei 85 cd/m², für Kontrastsehvermögen 0,032 cd/m², für Blendungsempfindlichkeit 0,1 cd/m²) Testzeichen mit unterschiedlichen Kontrastverhältnissen dargeboten, welche der Proband erkennen muss. Aus der kleinsten noch erkannten Größe wird auf die Sehschärfe bei der jeweiligen Umgebungshelligkeit geschlossen.

In der CA 2 438 109 A wird ein Gerät zur Bestimmung des Kontrastsehens beschrieben, bei dem auch der Pupillendurchmesser oder Aberationen des Auges gemessen werden können. Die WO 02/083078 A offenbart ein Verfahren zur optimalen Bestimmung einer vorzunehmenden Refraktionskorrektur, bei dem unter verschiedenen Bedingungen gemessene Wellenfrontaberationen mit statistischen Methoden bewertet werden.

Aus der US 2002/140902 A1 ist ein Verfahren zur Bestimmung einer optimalen Refraktionskorrektur bekannt, bei der gemessene Aberationsdaten mit einer eine Bildqualität repräsentierenden Metrik optimiert werden.

Die US 5 963 300 A beschreibt ein binokulares Aberometer, bei dem über zusätliche Messfunktionen geometrische Abmessungen des Auges bestimmbar sind.

### Aufgabe der Erfindung:

Aufgabe der Erfindung ist es, ein Verfahren und eine Einrichtung zur Bestimmung objektiver Parameter für die Sehfunktion des menschlichen Auges bei definierten Umgebungshelligkeiten anzugeben.

### Erfindung:

Diese Aufgabe wird erfindungsgemäß durch die Merkmale der unabhängigen Ansprüche gelöst. Bevorzugte Weiterentwicldungen sind in den abhängigen Ansprüchen beschrieben. Das erfindungsgemäße Verfahren und und die Einrichtung ermöglichen es bei definierten Umgebungshelligkeiten die Parameter der Sehfunktion zu bestimmen und gleichzeitig die sich bei der definierten Umgebungshelligkeit einstellende Pupillenweite sowie die Aberrationen des Auges zu messen.

Dadurch ist eine objektive Bewertung des Einflusses von Sehfehlern (Aberrationen) höherer Ordnung auf die Sehqualität möglich.

Neben der umfassenden Charakterisierung der Sehqualität ermöglich das Verfahren und die Einrichtung weiterhin Nachfolgemessungen, bei denen die gemessenen Aberrationen durch geeignete Maßnahmen kompensiert wurden.

Eine besonders bevorzugte Variante der Erfindung ergibt sich, wenn zur Kompensation der gemessenen Aberrationen höherer Ordnung (d.h. jenseits von Sphäre und Zylinder) - die der Pupillenweite entsprechend der Umgebungshelligkeit streng zugerordnet sind - geeignete optische Mittel (Phasenplatte, adaptiv optische Elemente eingeschwenkt) werden, und unter diesen Bedingungen eine Bestimmung der Sehfunktion erfolgt.

Die Bestimmung der Sehqualität bei kompensierten Aberrationen ist für die Beratung und Planung vor refraktiv-chirurgischen Eingriffen (preview-Funktion) von Bedeutung, und ermöglicht danaeben eine selektive Diagnostik von Störungen der neuronalen visuellen Funktionen.

### Beschreibung:

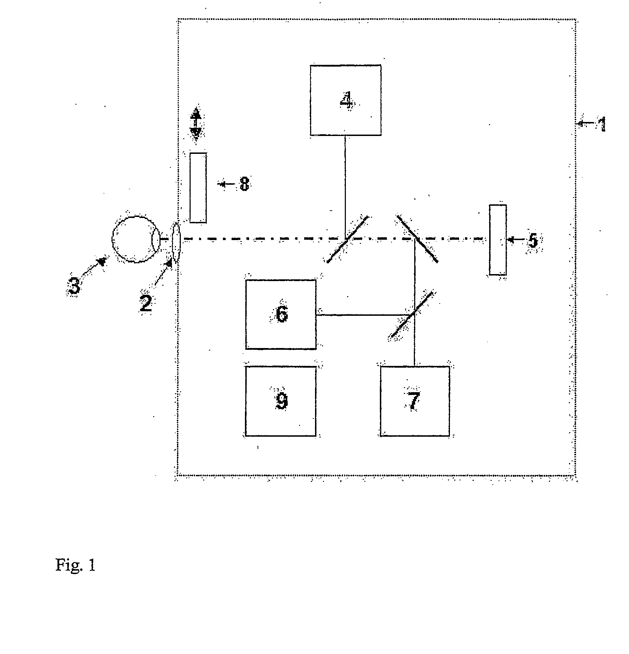

In Fig. 1 ist eine bevorzugte Ausführungsform der Erfindung schematisch dargestellt. Die Vorrichtung ist mit einer lichtdichten Umhüllung (1) umgeben, die einen monokularen oder vorzugsweise binokularen Einblick (2) zur Untersuchung des(der) Augen (3) besitzt. Zur Kontrolle und Einstellung einer definierten Helligkeit dient die Beleuchtungseinrichtung (4).

Bei frei wählbaren, unterschiedlichen Beleuchtungsverhälinissen werden den Auge(n) des Probanden Sehteste mit unterschiedlichen Kontrastverhältnissen (5) zur Prüfung der Sehfunktion dargeboten.

Mit den Meßsystemen (6) und (7) werden gleichzeitig der Pupillendurchmesser (6) und die Aberrationen (7) des Auges bei der eingestellten definierten Umgebungsbeleuchtung gemessen. Eine solche Einheit zur Bestimmung des Pupillendurchmessers (6) ist an sich vorbekannt und kann z.B. wie in DE 196 41 632 oder DE 196 49 542 beschrieben, aufgebaut sein und funktionieren. Die Einrichtung (7) zur Bestimmung der Aberrationen des Auges kann beispielsweise einen Wellenfrontsensor nach Hartmann-Shack enthalten, das Prinzip ist u. a. in Liang, et al., "Objective Measurement of Wave Aberrations of the Human Eye With the Use of a Hartmann-Shack Wave-front Sensor", J. Opt. Soc. Am. A., vol. 11, No. 7, pp. 1949-1957, (Juli 1994) beschrieben. Ebenso kann die Einrichtung (7) zur Bestimmung der Aberrationen des Auges ein Aberrometer sein, wie es z.B. als WASCA von der Firma Carl Zeiss Meditec AG hergestellt wird.

Mittels eines oder mehrer Kompensatoren (8) können Fehler des optischen Systems des Auges korrigiert werden, um das Sehvermögen bei unterschiedlichen Korrekturzuständen erfassen zu können. Die optischen Kompensatoren können einfache optische Elemente (Linsen), spezielle Phasenplatten oder aber adaptiv-optische Elemente sein. Im letzteren Falle wird das Ausgangssignal der Einheit zur Bestimmung des Pupillendurchmessers (6) und der Aberrationen (7) einer Rechner-und Steuereinheit (9) zugeführt, die aus den Daten die Steuerdaten für die Steuerung eines adaptiv-optischen Kompensators liefert und damit eine Korrektur des Einflusses der gemessenen Aberrationen ermöglicht.

Die Rechner- und Steuereinheit (9) ist darüberhinaus für die gesamte Ablaufsteuerung und Bedienerführung des Gesamtgerätes und seiner Teilfunktionen zuständig, sowie für die Erfassung, Verarbeitung, Darbietung und Ausgabe der Meßdaten zuständig und ermöglich darüberhinaus eine Datenkommunikation mit extremen Systemen.

Die Bestimmung der Sehqualität bei kompensierten Aberrationen ist für die Beratung und Planung vor refraktiv-chirurgischen Eingriffen (preview-Funktion) von großer Bedeutung. Derartige Messungen ermöglichen zudem eine Separation optisch bedingter Störungen (Aberrationen) im vorderen Augenabschnitt von krankhaften Veränderungen des hinteren Augenabschnittes.(Diabetische Retinopathie, Altersbedingte Makuladegenerationen, Glaukom). und erleichtert damit eine selektive Diagnostik von Störungen der neuronalen visuellen Funktionen.

Durch die gemeinsame Messung der Sehleistungen und der objektiven physikalischen Bedingungen für die Erreichung dieser Leistungen wird die Behandlungszeit reduziert. Durch die damit verbundene effektive und verlustfreie Verknüpfung der Meßdaten erhöht sich die Qualität des Befundungsergebnisses.

Die Realisierung der Erfindung ist nicht an das dargestellte Ausführungsbeispiel gebunden, fachmännische Weiterentwicklungen führen nicht zu einem Verlassen des Schutzbereiches der Patentansprüche.

## Patentansprüche

1. Verfahren zur Charakterisierung der Sehfunktion des Auges bei unterschiedlicher Umgebungshelligkeit, wobei
unter im Wesentlichen definierten Bedingungen der Umgebungshelligkeit zusätzlich zu Parametern der Sehfunktion vorzugsweise gleichzeitig sehfunktionsbestimmende Parameter wie die Pupillenweite und die Aberrationen des Auges erfasst werden, **dadurch gekennzeichnet, daß** Fehler des optischen Systems des Auges mittels eines oder mehrerer Kompensatoren kompensiert werden können, wobei die Kompensatoren über eine sphärische Korrektur hinaus eine Korrektur von zylindrischen Fehlern und/oder höheren Aberrationen bewirken.

2. Verfahren zur Charakterisierung der Sehfunktion nach Anspruch 1, wobei zur Kompensation bestehender Fehlsichtigkeiten entsprechende Korrekturgläser eingeschaltet werden.

3. Verfahren zur Charakterisierung der Sehfunktion nach Anspruch 2, wobei zur Kompensation einer etwaigen Nachtmyopie Minusgläser vorgeschaltet werden.

4. Verfahren zur Charakterisierung der Sehfunktion nach Anspruch 2, wobei zur Kompensation der gemessenen Aberrationen höherer Ordnung geeignete optische Mittel vor oder während der Messung der Sehfunktion eingeschwenkt werden.

5. Augenuntersuchungsgerät zur Charakterisierung der Sehfunktion des Auges (3), welches eine Einrichtung zur Bestimmung der Dämmerungssehschärfe und/oder Blendungsempfindlichkeit sowie eine Einrichtung zur Bestimmung des Pupillendurchmessers (6) und/oder der Aberrationen des Auges (7) aufweist, **gekennzeichnet dadurch, dass** weiterhin Kompensatoren (8) zur Korrektur von Fehlern des optischen Systems des Auges vorgesehen sind, wobei die Kompensatoren über eine sphärische Korrektur hinaus eine Korrektur von zylindrischen Fehlern und/oder höheren Aberrationen bewirken.

## Claims

1. Method for characterizing the visual function of the eye at different ambient brightness, in which, under substantially defined conditions of the ambient brightness, in addition to parameters of the visual function, preferably simultaneously, parameters determining the visual function such as the pupil width and the aberrations of the eye are detected, **characterized in that** errors of the optical system of the eye can be compensated by means of one or more compensators, the compensators effecting, beyond a spherical correction, a correction of cylindrical errors and/or higher aberrations.

2. Method for characterizing the visual function according to Claim 1, in which appropriate correcting lenses are interposed in order to compensate existing visual deficiencies.

3. Method for characterizing the visual function according to Claim 2, in which negative lenses are placed in front in order to compensate a possible night myopia.

4. Method for characterizing the visual function according to Claim 2, in which suitable optical means are pivoted in before or during the measurement of the visual function in order to compensate the measured aberrations of higher order.

5. Eye examination apparatus for characterizing the visual function of the eye (3), which has a device for determining the twilight visual acuity and/or glare sensitivity, as well as a device for determining the pupil diameter (6) and/or the aberrations of the eye (7), **characterized in that** compensators (8) are provided for correcting errors in the optical system of the eye, the compensators effecting, beyond a spherical correction, a correction of cylindrical errors and/or higher aberrations.

## Revendications

1. Procédé pour la caractérisation de la fonction visuelle de l'oeil dans diverses luminosités ambiantes, dans lequel dans des conditions définies pour l'essentiel de la luminosité ambiante, on enregistre en plus des paramètres de la fonction visuelle, de préférence simultanément des paramètres définis par la fonction visuelle comme la taille de la pupille et les aberrations de l'oeil, **caractérisé en ce que** des défauts du système optique de l'oeil peuvent être compensés au moyen d`un ou de plusieurs compensateurs, les compensateurs, au-delà d'une correction sphérique, opérant une correction de défauts cylindriques et/ou d'aberrations plus élevées.

2. Procédé pour la caractérisation de la fonction visuelle selon la revendication 1, dans lequel on met en oeuvre des verres correcteurs correspondants pour la compensation d'amétropie existante.

3. Procédé pour la caractérisation de la fonction visuelle selon la revendication 2, dans lequel des verres négatifs sont montés en amont pour la compensation d'une éventuelle myopie nocturne.

4. Procédé pour la caractérisation de la fonction visuelle selon la revendication 2, dans lequel des moyens optiques appropriés sont insérés avant ou pendant la mesure de la fonction visuelle pour la compensation des aberrations mesurées d'ordre plus élevé.

5. Appareil d'examen oculaire pour la caractérisation de la fonction visuelle de l'oeil (3) comportant un équipement pour la détermination de l'acuité visuelle crépusculaire et/ou de la sensibilité à l'éblouissement ainsi qu'un équipement pour la détermination du diamètre de la pupille (6) et/ou des aberrations de l'oeil (7), **caractérisé en ce que** des compensateurs (8) sont de plus prévus pour la correction de défauts du système optique de l'oeil, les compensateurs, au-delà d'un correction sphérique, opérant une correction de défauts cylindriques et/ou d'aberrations plus élevées.
